Europäisches Patentamt

European Patent Office

Office européen des brevets

(11) Publication number: **0 071 032**
**B1**

## (12) EUROPEAN PATENT SPECIFICATION

(45) Date of publication of patent specification: **02.05.85**

(21) Application number: **82105793.2**

(22) Date of filing: **29.06.82**

(51) Int. Cl.⁴: **C 07 C 43/04, C 07 C 41/06**

(54) **Process for the preparation of methyl tert-butyl ether and ethyl tert-butyl ether.**

(30) Priority: **28.07.81 IT 2319581**

(43) Date of publication of application:
**09.02.83 Bulletin 83/06**

(45) Publication of the grant of the patent:
**02.05.85 Bulletin 85/18**

(84) Designated Contracting States:
**AT BE DE FR GB NL SE**

(56) References cited:
**GB-A-2 043 065**
**GB-A-2 049 693**
**US-A-4 071 567**

(73) Proprietor: **EUTECO IMPIANTI S.p.A.**
**Via Grazioli 11**
**I-20161 Milano (IT)**

(73) Proprietor: **PETROFLEX INDUSTRIA E**
**COMERCIO S.A.**
**Rua Paraná s/n Campos Eliseos**
**Duque de Caxias, RJ (BR)**

(72) Inventor: **Baratella, Pietro**
**Via Leopardi 8**
**I-20099 Sesto San Giovanni Milano (IT)**
Inventor: **Maiorano, Giovanni**
**Via Assietta 31**
**I-20161 Milano (IT)**
Inventor: **Schwarz, Peter**
**Via G. Boni 41**
**I-20144 Milano (IT)**
Inventor: **Valtorta, Luigi**
**Via Grandi 3**
**I-20033 Desio Milano (IT)**

(74) Representative: **Jacobacci, Filippo et al**
**c/o JACOBACCI-CASETTA & PERANI S.p.A. Via**
**Alfieri 17**
**I-10121 Torino (IT)**

Courier Press, Leamington Spa, England.

## Description

The present invention relates to the preparation of methyl tert-butyl ether (MTBE) and ethyl tert-butyl ether (ETBE) by the reaction of isobutylene, contained in a $C_4$-hydrocarbon fraction, with methanol and ethanol respectively under the influence of acidic catalysts.

In the specification which follows, by alkyl tert-butyl ether, or more simply ether, is meant MTBE and ETBE and by alcohol is meant equally methanol and ethanol.

The alkyl tert-butyl ethers are products of great interest, particularly in relation to their use as anti-knock additives in various types of fuels.

According to the known art, the alkyl tert-butyl ethers are prepared by reacting isobutylene and alcohol, in the liquid phase, under the influence of acid catalysts, as described for example in US Patents Nos. 2,391,084, 2,480,940, 3,121,124 and 2,170,000 and in Belgian Patent No. 612,388. As a rule, a hydrocarbon fraction is used for this purpose which has four carbon atoms ($C_4$-fraction) and results from the pyrolysis of petroleum fractions which contain isobutylene together with linear butenes, butadiene and, to a lesser extent, butanes. The alcohol reacts selectively with the isobutylene in the $C_4$-fraction and the reaction moves relatively quickly towards its thermodynamic equilibrium at moderate temperatures and in the presence of effective catalysts, such as acid sulphonate ion-exchange resins.

Since one is dealing with an equilibrium reaction, the reaction mixture always contains unreacted reagents and various recourses have been proposed in the art, designed especially to improve the conversion of the isobutylene in the $C_4$-fraction supplied. More particularly, it has been felt necessary to convert the isobutylene to such a degree as to discharge an exhausted $C_4$-fraction with an isobutylene content of 1% by volume or less, which is usable directly in other industrial operations. In particular the said exhaustion of the isobutylene should be carried out in the absence or substantial absence of oligomerization of the isobutylene which would reduce the yield of the useful reaction products.

More favourable thermodynamic equilibrium conditions for the reaction may be achieved by using a large excess of the alcohol compared with the stoichiometric quantity required for the reaction with the isobutylene, the excess alcohol also having the additional function of inhibiting the oligomerization of the isobutylene which has already been mentioned. It is found, however, that even a considerable excess of the alcohol does not suffice to exhaust the isobutylene in the $C_4$-fraction to the desired extent, at least in the synthesis of ETBE in which the thermodynamic equilibrium is less favourable than for MTBE and in any case, a considerable excess of the alcohol makes the separation of the reaction products difficult due to the formation of azeotropic alcohol-ether mixtures.

It has also been proposed to carry out the etherification in two separate reaction stages, one with an excess of isobutylene, the other with an excess of the alcohol, or in two stages in series, with separation of the ether produced downstream of each stage, as described for example in Belgian Patents Nos. 829,303 and 829,299. These methods of operation are not free from disadvantages. Indeed, in the first case mentioned, oligomerization of the isobutylene occurs in the stage which is carried out with an excess of this reagent, while in the second case mentioned, although it is possible to achieve a satisfactory conversion of the isobutylene in the synthesis of MTBE, there are complications resulting from the fact that, with two reaction stages, the distillation treatment is duplicated.

It has also been proposed to carry out the etherification reaction of methanol in the presence of a reduced amount of impoverished $C_4$-hydrocarbon cut or of inert aliphatic $C_5$- or $C_6$-hydrocarbon to facilitate the successive azeotropic separation of unconverted methanol, as described in GB Nos. 2,043,065 and 2,049,693.

While these processes are effective in ameliorating the separation and of consequence the purity of obtained methyl tert-butyl ether, they did not result in improving the conversion yield of methanol nor in reducing the unconverted isobutylene in the reaction mixture below the 1% by vol.

The object of the present invention is to overcome the disadvantages mentioned above with regard to the process for the preparation of alkyl tert-butyl ethers by the reaction of isobutylene with alcohols, under the influence of acidic catalysts. More particularly, the object of the present invention is to provide a process for the preparation of alkyl tert-butyl ethers by the reaction of alcohols with isobutylene in a $C_4$-hydrocarbon fraction, which allows improved thermodynamic equilibrium conditions to be achieved, resulting in better conversions of the isobutylene in the $C_4$-fraction supplied into ethers. In the preferred embodiment of the process which is the subject of the present invention, the isobutylene content of the exhausted $C_4$-fraction is reduced to values equal to or less than about 1% by volume, the reaction being effected in a simple reaction system, with a quantity of alcohol equal to, or approximately equal to, the stoichiometric quantity required for the reaction with the isobutylene and in the absence, or substantial absence, of oligomerization of the isobutylene.

The present invention is based essentially on the finding that the thermodynamic equilibrium of the reaction between isobutylene and an alcohol, in the liquid phase, is influenced by the inert aliphatic hydrocarbon medium in which the reaction occurs, in the sense that, other conditions being equal, the equilibrium is more favourable the greater the degree of dilution of the medium. Through this behaviour, it may be supposed that the behaviour of the reaction system is displaced from the ideal, particularly with regard to the alcohol, the coefficient of activity of which appears to increase considerably with the increase in the degree of dilution of the aliphatic-hydrocarbon medium in which the reaction is carried out. In each case, whatever the explanation of the phenomenon, the fact is that the course of the etherification in an aliphatic hydrocarbon medium diluted to a greater degree than given by the inert hydrocarbons in the $C_4$-fraction of the feed, allows the thermodynamic equilibrium to be displaced in favour of the reaction products, which allows the possibility of improving the conversion of the isobutylene, in a simple reaction system and with a quantity of alcohol equal to or approximately equal to the stoichiometric quantity.

Accordingly, according to the process of the present invention methyl tert-butyl ether or ethyl tert-butyl ether is prepared by the selective reaction of methanol or ethanol respectively with isobutylene contained in a $C_4$-hydrocarbon fraction, in the liquid phase, in the presence of acid catalysts, with a molar ratio of the alcohol to the isobutylene of from 1:1 to 1.25:1, at a temperature of from 40° to 100°C and at a pressure of from 15 to 40 bars, the reaction medium being diluted by an aliphatic hydrocarbon or mixtures of aliphatic hydrocarbons which are liquid under the operating conditions and inert to the other constituents of the reaction medium and which are the recycle product obtained from the reaction mixture after separation of at least the formed ether, characterised in that the recycle product is fed to the reaction medium in an amount to maintain, at the equilibrium, a molar ratio of aliphatic hydrocarbon with the formed ether higher than 7:1.

In the preferred embodiment, a quantity of inert aliphatics hydrocarbon is supplied which is such as to reduce the isobutylene content in the exhausted $C_4$-fraction to values equal to or less than about 1% by volume.

The $C_4$-hydrocarbon fractions which are subjected to the process of the present invention generally result from the thermal or catalytic pyrolysis of suitable petroleum fractions and, together with the isobutylene contain variable quantities of linear butenes, butadiene and butanes. The isobutylene content of the said $C_4$-fractions may generally vary from 14 to 55% by volume.

The alcohols used for the formation of the respective ethers are methanol and ethanol. It is convenient for these alcohols to be anhydrous, or at least for their water content to be maintained at values less than about 0.3% by weight, so as to avoid, or at least minimize, the formation of by-products such as tertiary butyl alcohols.

According to the present invention, the molar ratio of the alcohol to the isobutylene in the feed is maintained at a value of from 1/1 to 1.25/1, preferred values being of the order of 1.05/1 to 1.1/1. It has been found that under these conditions the formation of oligomers of isobutylene is avoided or at least minimized. The use of alcohol/isobutylene ratios above the maximum indicated does not result in any substantial advantages with regard to the conversion of the isobutylene and introduces operative complications into the reaction system.

The etherification reaction is carried out in the presence of acid catalysts. Although any catalysts which can favour the reaction between the alcohol and isobutylene are useful for this purpose, according to the present invention the preferred catalysts are solid, acid sulphonate ion-exchange resins. Examples of these resins are commercially available under the following trade marks: Amberlite IR-1, Amberlite IR-100, Nalcite MX, Dowex 50, Nalcite HCR and Amberlyst-15. Of the sulphonated exchange resins, those particularly preferred have a matrix of polystyrene cross-linked with divinylbenzene and are in the form of granules with sizes of from 16 to 50 mesh.

Although the etherification temperatures could vary within a wide range, such as from the ambient temperature up to a maximum which depends on the thermal stability of the catalyst, according to the present invention, the reaction is carried out in a range of from 40 to 100°C, preferably 45 to 100°C so as to achieve a good reaction rate while preventing damage to the resinous catalyst where such a catalyst is chosen.

The etherification pressures are at least those required to maintain the reaction medium in the liquid phase under the reaction conditions. According to the invention, given the temperature range indicated, suitable pressures for the purpose lie within the range 15 to 40 bar.

The reaction times are those required to achieve reaction conditions equal to or approximately equal to those of thermodynamic equilibrium of the reactive system.

These conditions are, for example, achieved when the reaction is carried out continuously at the temperatures indicated above, the reagent mixture being brought into contact with the acid sulphonate exchange resin disposed in the form of a fixed bed, with a space velocity of from 3 to 20 hour$^{-1}$. The space velocity in hour$^{-1}$ is the reciprocal of the contact time expressed in hours.

The basic aspect of the present invention is that the etherification is carried out in an inert aliphatic hydrocarbon medium which is diluted to a degree such as to achieve more favourable thermodynamic equilibrium conditions, and, by definition, achieve a higher yield of the desired ether, and to discharge an exhausted $C_4$-fraction with a small isobutylene content, preferably with an isobutylene content equal to or less than about 1% by volume. Such an exhausted $C_4$-fraction is in fact usable directly in industrial processes, for example, for the preparation of maleic-anhydride or methyl ethyl ketone. In the preferred embodiment, the exhausted $C_4$-fraction resulting from the same or another etherification reaction of the inventive process is used as the diluent and is recycled to the reaction medium so as to create the desired dilution conditions. By the exhausted fraction is meant the $C_4$-fraction which remains after the reaction between the alcohol and the isobutylene and from which at least the ether produced has been separated.

The recycled, exhausted $C_4$-fraction may contain residual isobutylene, although to a smaller extent than in the fresh $C_4$-fraction, and generally not more than 1% by volume. It is necessary to take account of this residual isobutylene introduced into the reaction medium in order to calculate the ratio of the alcohol to the isobutylene correctly.

In practice, the higher the degree of dilution of the inert, aliphatic-hydrocarbon medium in which the etherification reaction is carried out, the greater is the conversion of the isobutylene to the respective ether, other conditions being equal.

If the reaction is carried out continuously within the range of preferred conditions mentioned above, it is possible to reduce the isobutylene content in the exhausted $C_4$-fraction to values of the order of 1% by volume, the reaction medium being diluted to give a molar ratio of the total quantity of the inert, aliphatic hydrocarbons to the ether formed of the order of 7/1 to 8/1 in the case of MTBE and to values of the order of 15/1 to 23/1 in the case of ETBE, under conditions equal to or approximately equal to equilibrium conditions. By the total quantity of inert, aliphatic hydrocarbons is meant the sum of the number of moles of hydrocarbon introduced in the fresh $C_4$-fraction and those introduced intentionally into the system, for example, by means of the recycled, exhausted $C_4$-fraction. In order to achieve a greater exhaustion of the isobutylene, the ratio mentioned above is increased proportionally, account being taken of the fact that no significant advantages are achieved with values of this ratio greater than 10/1 to 12/1 in the case of MTBE and greater than 25/1 to 30/1 in the case of ETBE.

The reaction being carried out according to the process of the present invention it is possible to prepare MTBE from methanol and a $C_4$-fraction containing isobutylene and to discharge an exhausted $C_4$-fraction with an isobutylene content equal to or less than about 1% by volume, in a single reaction stage, resulting in considerable advantages and simplifications. It is also possible to prepare ETBE by the present invention, an exhausted $C_4$-fraction being discharged with an isobutylene content equal to or less than about 1% by volume. However, given the less favourable equilibrium conditions, it is convenient to prepare ETBE in a single reaction stage only when a fresh $C_4$-fraction is used with a low isobutylene content, for example equal to or less than about 20% by volume.

With isobutylene contents in the $C_4$-fraction greater than the last value mentioned, ETBE can conveniently be prepared in two stages in series and, in this case, the dilution with inert hydrocarbons is carried out in the reaction stage in which the isobutylene is exhausted. Obviously it is also possible to prepare MTBE in a two-stage process with dilution in the stage in which the isobutylene is exhausted. However, this method of operation does not result in significant advantages with regard to the conversion of the isobutylene and is more complicated to operate than the single stage process.

The experimental examples which follow are illustrative and non-limiting of the invention.

Example 1 (comparison)

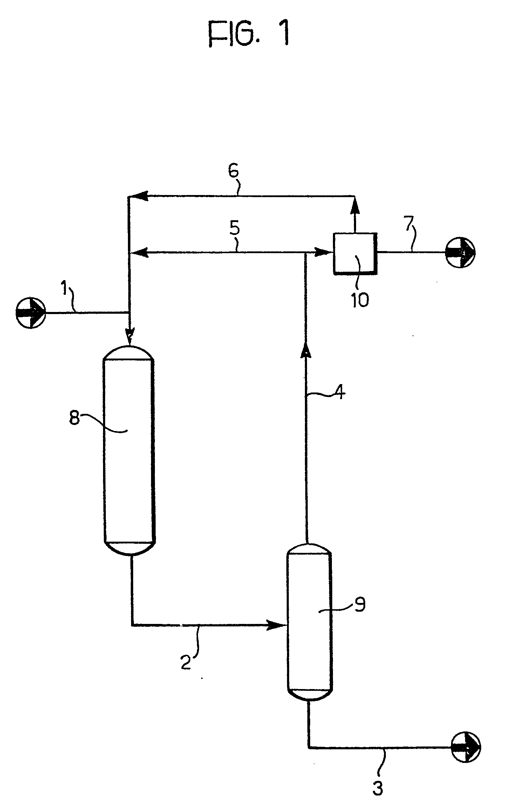

MTBE is prepared by bringing methanol into contact with isobutylene contained in a $C_4$-fraction, the reaction being carried out continuously in a single reaction stage in accordance with the diagram of Figure 1.

For this purpose methanol with a 99.6% titre by weight and a water content of less than 0.3% by weight is used together with a $C_4$-fraction, resulting from the thermal pyrolysis of a petroleum fraction, having the following percentage molar composition:

| | | |
|---|---|---|
| isobutylene | : | 45.0% |
| linear butenes | : | 41.6% |
| butadiene | : | 0.4% |
| butanes | : | 13.0% |

In the following description, the combination of the linear butenes, butadiene and butanes will be indicated by "inert compounds".

With reference to Figure 1, a fresh reagent mixture is fed into the reactor 8 through the line 1 with the following composition:

| | | |
|---|---|---|
| isobutylene | : | 1.343 Kg/hour |
| inert compounds | : | 1.646 Kg/hour |
| methanol | : | 0.734 Kg/hour |

0.109 Kg/hour of recycled methanol is also fed to the reactor 8 through the line 6. Thus the molar ratio between the methanol and the isobutylene in the feed is equal to 1.1/1. In this test none of the exhausted $C_4$-fraction is recycled.

The reactor 8 is an elongate tubular reactor provided with heat-exchange means and containing the sulphonated ion-exchange resin Amberlyst 15 in the form of granules with sizes of from 16 to 50 mesh, disposed in the form of a fixed bed. The etherification is carried out in this reactor in the liquid phase, at about 55°C, at a pressure of 20 bar, with a space velocity of the reagent mixture of 5 $hour^{-1}$ and the reaction mixture is discharged through the line 2 with the following composition:

| | | |
|---|---|---|
| isobutylene | : | 0.070 Kg/hour |
| inert compounds | : | 1.646 Kg/hour |
| methanol | : | 0.116 Kg/hour |
| MTBE | : | 2.000 Kg/hour |

This mixture is distilled in the column 9 with a temperature of 118°C at the foot and 53°C at the head, under a pressure of 5.5 bar measured at the head. Under these conditions, MTBE is separated at the foot of the column (purity about 99%) and is recovered through the line 3. The products at the head of the column recovered through the line 4, are treated in unit 10 so as to recover the residual methanol which is recycled to the reactor 8 through the line 6. The exhausted $C_4$-fraction discharged through the line 7 contains about 4% by volume of isobutylene.

The yield of MTBE, evaluated on the isobutylene supplied, is thus 94.8%.

Example 2 (comparison)

This is carried out in a manner similar to Example 1, a fresh reagent mixture being fed to the reactor 8 through the line 1 with the following composition:

| | | |
|---|---|---|
| isobutylene | : | 1.343 Kg/hour |
| inert compounds | : | 1.646 Kg/hour |
| methanol | : | 0.755 Kg/hour |

To the reactor is also supplied 0.037 Kg/hour of recycled methanol (line 6) and a recycled $C_4$-fraction, free from ether, through the line 5, with the following composition:

| | | |
|---|---|---|
| isobutylene | : | 0.042 Kg/hour |
| inert compounds | : | 2.043 Kg/hour |
| methanol | : | 0.078 Kg/hour |

Thus the molar ratio of the methanol to the isobutylene in the feed is 1.1/1.

In the reactor 8 the reaction is carried out in a similar manner to Example 1, the height of the catalytic bed being varied so as to achieve a space velocity of 5 hour$^{-1}$; a reaction mixture is discharged with the following composition.

| | | |
|---|---|---|
| isobutylene | : | 0.075 Kg/hour |
| inert compounds | : | 3.699 Kg/hour |
| methanol | : | 0.135 Kg/hour |
| MTBE | : | 2.058 Kg/hour |

Thus the molar ratio of the total quantity of inert hydrocarbons to the MTBE, under approximately equilibrium conditions, is 2.8/1.

The reaction mixture is passed through the line 2 to the distillation column 9 where MTBE separates at the foot (99% pure) and is recovered through the line 3. The products at the head recovered through the line 4, are partly recycled to the reactor 8 through the line 5 and partly treated in unit 10 to recover the residual methanol and this part is recycled to the reactor through the line 6. The exhausted $C_4$-fraction discharged through the line 7 contains about 2% by volume of isobutylene.

The yield of MTBE is thus about 97.5%.

Example 3

This is carried out in a similar manner to Example 1, a fresh reagent mixture being fed to the reactor 8 through the line 1, with the following composition:

| | | |
|---|---|---|
| isobutylene | : | 1.343 Kg/hour |
| inert compounds | : | 1.646 Kg/hour |
| methanol | : | 0.764 Kg/hour |

The reactor is also fed (line 6) with 0.017 Kg/hour of recycled methanol and, through line 5, with a recycled $C_4$-fraction, free from ether, having the following composition:

| | | |
|---|---|---|
| isobutylene | : | 0.083 Kg/hour |
| inert compounds | : | 8.177 Kg/hour |
| methanol | : | 0.115 Kg/hour |

Thus the molar ratio of the methanol to the isobutylene in the feed is 1.1/1.

In the reactor 8 the reaction is carried out in a similar manner to Example 1, the height of the catalytic bed being varied so as to achieve a space velocity of 5 hour$^{-1}$ and a reaction mixture being discharged with the following composition:

| | | |
|---|---|---|
| isobutylene | : | 0.099 Kg/hour |
| inert compounds | : | 9.823 Kg/hour |
| methanol | : | 0.138 Kg/hour |
| MTBE | : | 2.084 Kg/hour |

Thus the molar ratio of the total quantity of inert hydrocarbons to the MTBE, under approximately equilibrium conditions, is about 7.4/1. The reaction mixture is treated in a similar manner to that described in Example 2 to obtain MTBE (purity about 99%) at the foot of the column 9, an exhausted $C_4$-fraction containing about 1% by volume of isobutylene being discharged through the line 7.

The yield of MTBE is thus about 98.8%.

Example 4

This is carried out in a similar manner to Example 1, a fresh reagent mixture being fed to the reactor 8 through the line 1 with the following composition:

| | | |
|---|---|---|
| isobutylene | : | 1.343 Kg/hour |
| inert compounds | : | 1.646 Kg/hour |
| methanol | : | 0.766 Kg/hour |

To the reactor is also fed (line 6) 0.025 Kg/hour of recycled methanol, and through the line 5, a recycled C₄-fraction free from ether and having the following composition:

| | | |
|---|---|---|
| isobutylene | : | 0.104 Kg/hour |
| inert compounds | : | 12.970 Kg/hour |
| methanol | : | 0.140 Kg/hour |

Thus the molar feed ratio of the methanol to the isobutylene in the feed is 1.1/1.

In the reactor 8 the reaction is carried out in a similar manner to Example 1, the height of the catalytic bed being varied so as to achieve a space velocity of 5 hour⁻¹ and a reaction mixture being discharged with the following composition:

| | | |
|---|---|---|
| isobutylene | : | 0.117 Kg/hour |
| inert compounds | : | 14.614 Kg/hour |
| methanol | : | 0.158 Kg/hour |
| MTBE | : | 2.090 Kg/hour |

Thus the molar ratio of the total quantity of inert hydrocarbons to the MTBE, under approximately equilibrium conditions, is about 11/1.

The reaction mixture is treated in a similar manner to that described in Example 2, MTBE (purity about 99%) being obtained at the foot of the column 9 and an exhausted C₄-fraction being discharged through the line 7 with a content of about 0.8% by volume of isobutylene. The yield of MTBE is about 99%.

In all the examples described, only traces of oligomers of isobutylene were present in the etherification products.

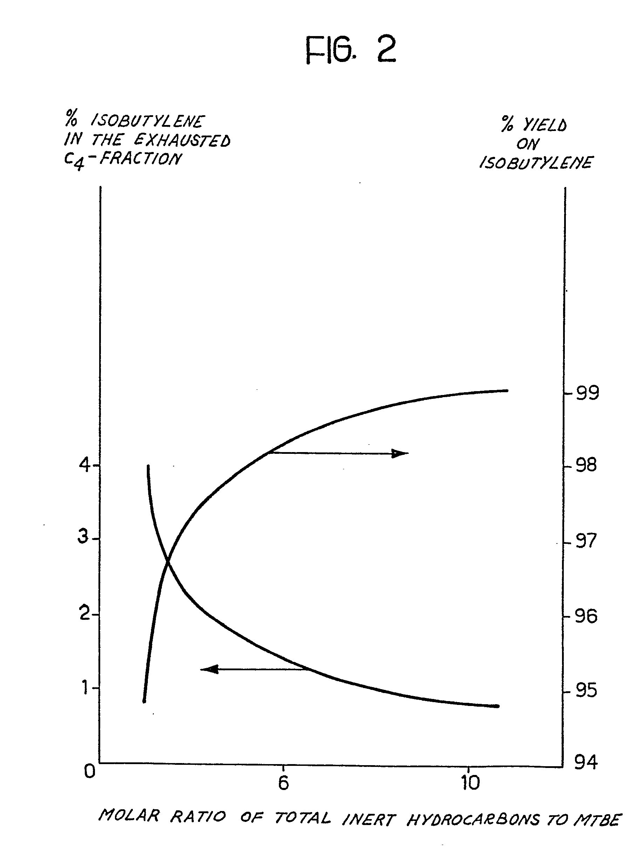

Figure 2 shows graphically the course of the reactions of Examples 1 to 4, the molar ratio of the total quantity of inert aliphatic hydrocarbons to the MTBE formed, under approximately equilibrium conditions, being given on the abscissa, as a function of the percentage by volume isobutylene in the exhausted C₄-fraction discharged and of the yield of MTBE expressed as a molar percentage of the isobutylene feed.

Example 5 (comparison)

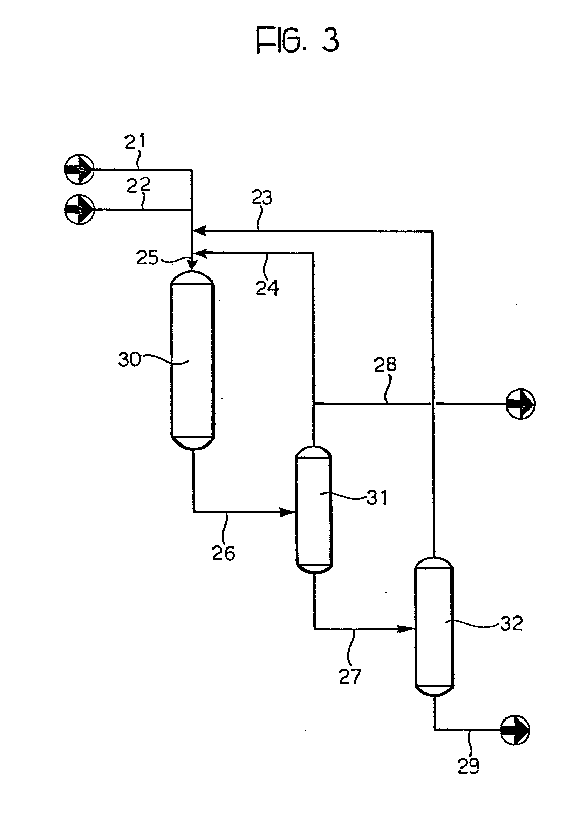

ETBE is prepared by bringing ethanol into contact with isobutylene under continuous conditions, in a single reaction stage, in accordance with the diagram of Figure 3.

For this purpose ethanol, with a titre of 99.6% by wt. and with a water content of less than 0.3%, by wt. is used together with a C₄-fraction which results from the catalytic pyrolysis of a petroleum fraction having the following molar percentage composition:

| | | |
|---|---|---|
| isobutylene | : | 20.0% |
| linear butenes | : | 28.2% |
| butadiene | : | 0.8% |
| butanes | : | 51.0% |

In the following description, the combination of the linear butenes, butadiene and butanes will be indicated by the term "inert compounds".

With reference to Figure 3, 0.902 Kg/hour of ethanol is fed to the reactor 30 through the line 21 and 7.351 Kg/hour of the fresh C₄-hydrocarbon fraction indicated above is fed through the line 22.

To the reactor 30, through line 23, is also fed a recycle flow having the following composition:

| | | |
|---|---|---|
| ethanol | : | 0.344 Kg/hour |
| ETBE | : | 0.800 Kg/hour |

Thus the flow entering the reactor 30 through the line 25 has the following overall composition:

| | | |
|---|---|---|
| isobutylene | : | 1.444 Kg/hour |
| inert compounds | : | 5.907 Kg/hour |
| ethanol | : | 1.245 Kg/hour |
| ETBE | : | 0.800 Kg/hour |

with an ethanol/isobutylene molar ratio of 1.05/1.

The reactor 30 is similar to that described in Example 1 and the etherification is carried out in the said reactor with the use of the catalyst Amberlyst 15 in the form of a fixed bed, with the reaction system in the liquid phase, at about 70°C, under a pressure of 20 bar and with a space velocity of the reagent mixture of about 10 hour⁻¹; the reaction mixture is discharged through the line 26 with the following composition:

| | | |
|---|---|---|
| isobutylene | : | 0.346 Kg/hour |
| inert compounds | : | 5.907 Kg/hour |
| ethanol | : | 0.344 Kg/hour |
| ETBE | : | 2.800 Kg/hour |

This mixture is subjected to a first distillation in the column 31 which is operated with a temperature at the foot of 112°C and at the head of 23°C, and at a pressure of 3 bar measured at the head.

Under these conditions a flow is separated at the head of the column and is discharged through the line 28 with the following composition:

| | | |
|---|---|---|
| isobutylene | : | 0.346 Kg/hour |
| inert compounds | : | 5.907 Kg/hour |

In this test no recycling of the exhausted C₄-fraction is effected. At the foot of the column 31 a flow is recovered through the line 27, consisting essentially of ethanol and ETBE, which is subjected to distillation in the column 32 operated with a temperature at the foot of 108°C and at the head of 98°C, and at a pressure of 3 bar measured at the head. Under these conditions ETBE (purity about 99%) is separated at the foot and recovered through the line 29 and a flow of ethanol and ETBE is recovered at the head and recycled through the line 23.

The quantity of isobutylene present in the exhausted $C_4$-fraction discharged through the line 28 is about 5.6% by volume.

The yield of ETBE, evaluated on the isobutylene in the feed is thus about 76%.

Example 6 (comparison)

This is carried out like Example 5, there being supplied to the reactor 30: 0.936 Kg/hour of ethanol (line 21) and 7.351 Kg/hour of the fresh $C_4$-fraction described in Example 5 (line 22). To the reactor is also fed, through the line 23, a recycle flow constituted by:

ethanol                  :  0.364 Kg/hour

ETBE                     :  0.848 Kg/hour

and a recycle flow through the line 24 constituted by the exhausted $C_4$-fraction with the composition:

isobutylene              :  0.063 Kg/hour

inert compounds          :  1.230 Kg/hour

The total flow fed to the reactor 30 through the line 25 thus has the following composition:

isobutylene              :  1.508 Kg/hour

inert compounds          :  7.136 Kg/hour

ethanol                  :  1.300 Kg/hour

ETBE                     :  0.848 Kg/hour

with an ethanol/isobutylene molar ratio of 1.05/1.

The reactor 30 is operated as in Example 5, the height of the catalyst bed being varied so as to achieve a space velocity of 10 $hour^{-1}$ and a reaction mixture is discharged from the reactor through the line 26 with the following composition:

isobutylene              :  0.367 Kg/hour

inert compounds          :  7.136 Kg/hour

ethanol                  :  0.363 Kg/hour

ETBE                     :  2.925 Kg/hour

Thus the molar ratio of the total quantity of inert hydrocarbons to the ETBE under approximately equilibrium conditions is about 4.3/1.

This reaction mixture is distilled in the columns 31 and 32 under conditions similar to those of Example 5 and ETBE is discharged through the line 28 with a purity of about 99% and an exhausted $C_4$-fraction is discharged through the line 28 with an isobutylene content of about 5% by volume.

The yield of ETBE is about 79%.

Example 7 (comparison)

This is carried out like Example 5, there being fed to the reactor 30: 0.988 Kg/hour of ethanol (line 21) and 7.351 Kg/hour of the fresh $C_4$-fraction described in Example 5 (line 22). To the reactor is also fed, through the line 23, a recycled flow constituted by:

ethanol                  :  0.395 Kg/hour

ETBE                     :  0.924 Kg/hour

and, through the line 24, a recycle flow constituted by the exhausted $C_4$-fraction with the composition:

isobutylene              :  0.161 Kg/hour

inert compounds          :  3.950 Kg/hour

The total flow fed to the reactor 30 through the line 25 thus has the following composition:

isobutylene              :  1.606 Kg/hour

inert compounds          :  9.857 Kg/hour

ethanol                  :  1.384 Kg/hour

ETBE                     :  0.924 Kg/hour

with an ethanol/isobutylene molar ratio of 1.05/1.

The reactor 30 is operated as in Example 5, the height of the catalyst bed being varied so as to achieve a space velocity of 10 $hour^{-1}$ and a reaction mixture is discharged from the reactor through the line 26 with the following composition:

isobutylene              :  0.402 Kg/hour

inert compounds          :  9.857 Kg/hour

ethanol                  :  0.395 Kg/hour

ETBE                     :  3.116 Kg/hour

Thus the molar ratio of the total quantity of inert hydrocarbons to the ETBE under approximately equilibrium conditions is about 5.6/1.

This reaction mixture is distilled in the columns 31 and 32 under conditions similar to those of Example 5 and ETBE is discharged through the line 29 with a purity of about 99% and the exhausted $C_4$-fraction is discharged through the line 28 with an isobutylene content of about 4% by volume.

The yield of ETBE is about 83.3%.

Example 8

This is carried out as in Example 5, there being fed to the reactor 30: 1.039 Kg/hour of ethanol (line 21) and 7.351 Kg/hour of the fresh $C_4$-fraction described in Example 5 (line 22). To the reactor is also fed, through the line 23, a recycle flow constituted by:

| | | |
|---|---|---|
| ethanol | : | 0.433 Kg/hour |
| ETBE | : | 1.010 Kg/hour |

and, through the line 24, a recycle flow constituted by the exhausted $C_4$-fraction with the composition:

| | | |
|---|---|---|
| isobutylene | : | 0.263 Kg/hour |
| inert compounds | : | 8.703 Kg/hour |

The total flow fed to the reactor 30 through the line 25 thus has the following composition:

| | | |
|---|---|---|
| isobutylene | : | 1.707 Kg/hour |
| inert compounds | : | 14.609 Kg/hour |
| ethanol | : | 1.633 Kg/hour |
| ETBE | : | 1.010 Kg/hour |

with an ethanol/isobutylene molar ratio of 1.05/1.

The reactor 30 is operated as in Example 5, the height of the catalyst bed being varied so as to achieve a space velocity of 10 hour$^{-1}$ and a reaction mixture is discharged from the reactor through the line 26 with the following composition:

| | | |
|---|---|---|
| isobutylene | : | 0.442 Kg/hour |
| inert compounds | : | 14.609 Kg/hour |
| ethanol | : | 0.433 Kg/hour |
| ETBE | : | 3.316 Kg/hour |

Thus the molar ratio of the total quantity of inert hydrocarbons to the ETBE under approximately equilibrium conditions is about 7.8/1.

This reaction mixture is distilled in the columns 31 and 32 under conditions similar to those of Example 5 and ETBE is discharged through the line 29 with a purity of about 99% and the exhausted $C_4$-fraction is discharged through the line 28 with an isobutylene content of about 3% by volume.

The yield of ETBE is about 87.6%.

Example 9

This is carried out as in Example 5, there being fed to the reactor 30: 1.089 Kg/hour of ethanol (line 21) and 7.351 Kg/hour of the fresh $C_4$-fraction described in Example 5 (line 22). To the reactor is also fed, through the line 23, a recycle flow constituted by:

| | | |
|---|---|---|
| ethanol | : | 0.476 Kg/hour |
| ETBE | : | 1.112 Kg/hour |

and, through the line 24, a recycle flow constituted by the exhausted $C_4$-fraction, with the composition:

| | | |
|---|---|---|
| isobutylene | : | 0.371 Kg/hour |
| inert compounds | : | 18.607 Kg/hour |

The total flow fed to the reactor 30 through the line 25 thus has the following composition:

| | | |
|---|---|---|
| isobutylene | : | 1.816 Kg/hour |
| inert compounds | : | 24.514 Kg/hour |
| ethanol | : | 1.567 Kg/hour |
| ETBE | : | 1.112 Kg/hour |

with an ethanol/isobutylene molar ratio of 1.05/1.

The reactor 30 is operated as in Example 5, the height of the catalyst bed being varied so as to achieve a space velocity of 10 hour$^{-1}$ and a reaction mixture is discharged from the reactor through the line 26 with the following composition:

| | | |
|---|---|---|
| isobutylene | : | 0.489 Kg/hour |
| inert compounds | : | 24.514 Kg/hour |
| ethanol | : | 0.476 Kg/hour |
| ETBE | : | 3.527 Kg/hour |

Thus the molar ratio of the total quantity of inert hydrocarbons to the ETBE under approximately equilibrium conditions is about 12.3/1.

This reaction mixture is distilled in the columns 31 and 32 under conditions similar to those of Example 5 and ETBE is discharged through the line 29 with a purity of about 99% and the exhausted $C_4$-fraction is discharged through the line 28 with an isobutylene content of about 2% by volume.

The yield of ETBE is about 91.8%.

Example 10

This is carried out as in Example 5, there being fed to the reactor 30: 1.138 Kg/hour of ethanol (line 21) and 7.351 Kg/hour of the fresh $C_4$-fraction described in the Example 5 (line 22). To the reactor is also fed, through the line 23, a recycle flow constituted by:

| | | |
|---|---|---|
| ethanol | : | 0.499 Kg/hour |
| ETBE | : | 1.232 Kg/hour |

and, through the line 24, a recycle flow constituted by the exhausted $C_4$-fraction with the composition:

isobutylene : 0.488 Kg/hour

inert compounds : 49.378 Kg/hour

The total flow fed to the reactor 30 through the line 25 thus has the following composition:

isobutylene : 1.932 Kg/hour

inert compounds : 55.285 Kg/hour

ethanol : 1.637 Kg/hour

ETBE : 1.232 Kg/hour

with an ethanol/isobutylene molar ratio of 1.03/1.

The reactor 30 is operated as in Example 5, the height of the catalyst bed being varied so as to achieve a space velocity of 10 hour$^{-1}$ and a reaction mixture being discharged from the reactor through the line 26 with the following composition:

isobutylene : 0.546 Kg/hour

inert compounds : 55.285 Kg/hour

ethanol : 0.499 Kg/hour

ETBE : 3.756 Kg/hour

Thus the molar ratio of the total quantity of inert hydrocarbons to the ETBE under approximately equilibrium conditions is about 26.2/1.

This reaction mixture is distilled in the columns 31 and 32 under conditions similar to those of Example 5 and ETBE is discharged through the line 29 with a purity of about 99% and the exhausted C$_4$-fraction is discharged through the line 28 with an isobutylene content of about 1% by volume.

The yield of ETBE is about 96%.

In all the Examples 5 to 10 described, oligomers of isobutylene were present only in traces in the etherification products.

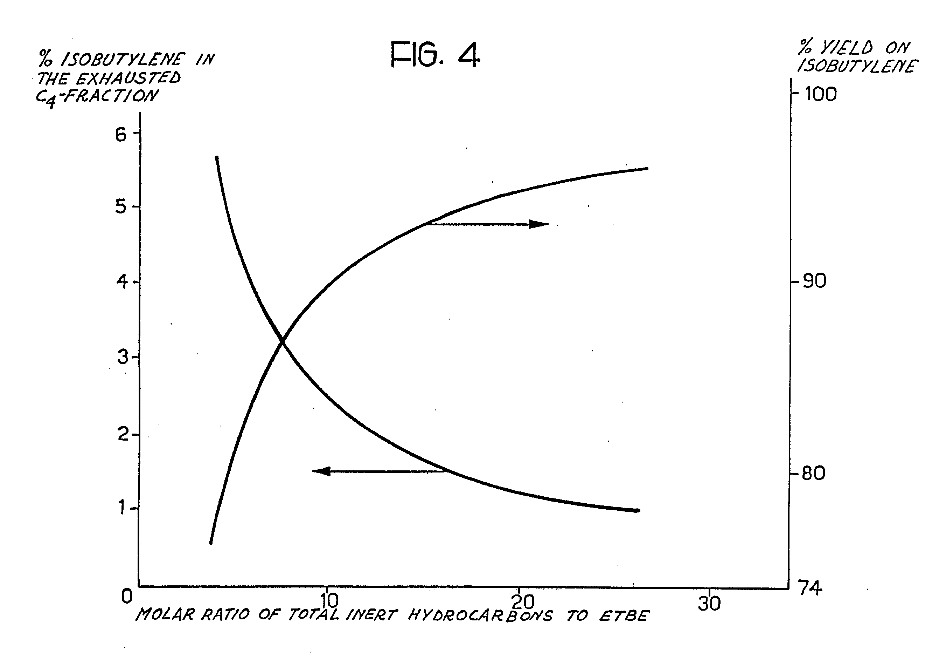

Figure 4 shows graphically the course of the reactions of the Examples 5 to 10, the molar ratio of the total quantity of inert, aliphatic hydrocarbons to the ETBE formed under approximately equilibrium conditions being shown on the abscissa as a function of the precentage volume of isobutylene in the exhausted C$_4$-fraction discharged and the yield of ETBE expressed as a molar percentage of the isobutylene supplied.

Example 11

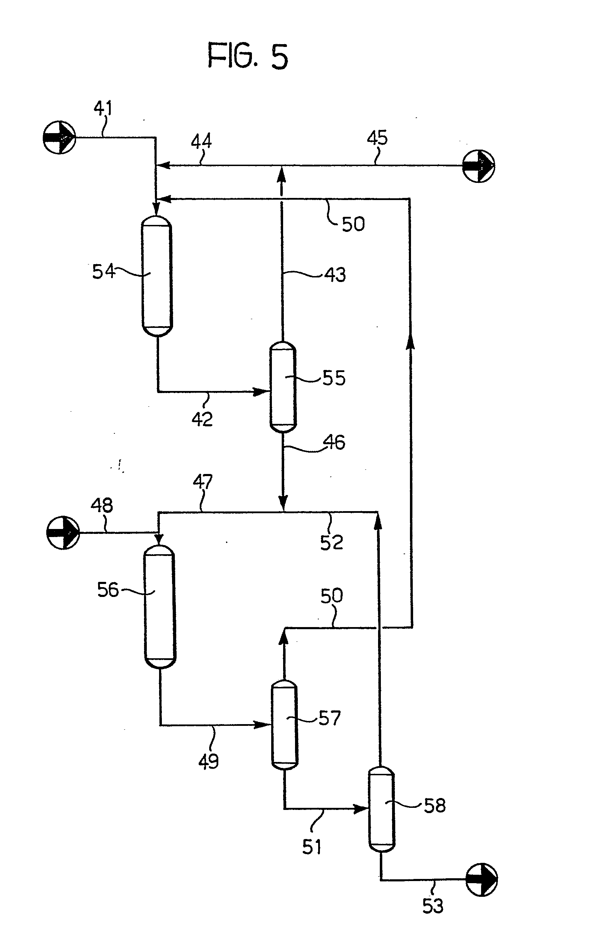

ETBE is prepared by bringing ethanol into contact with isobutylene continuously in a two-stage reaction according to the scheme of Figure 5.

For this purpose, ethanol with a titre of 99.6% by wt. and a water content of less than 0.3% by wt. is used together with the fresh C$_4$-fraction of Example 1.

With reference to Figure 5, the fresh C$_4$-fraction is fed to the reactor 56 of the first stage, through the line 48, in quantities of 2.458 Kg/hour, together with a flow, through the line 47, having the following composition:

ethanol : 1.014 Kg/hour

ETBE : 1.136 Kg/hour

The reactor 56 is operated with the Amberlyst 15 catalyst in the form of a fixed bed, with the reagents in the liquid phase, at about 70°C, under a pressure of 20 bar, and with a space velocity of the reagent mixture of approximately 10 hour$^{-1}$ and a reaction mixture is discharged through the line 49 with the following composition:

isobutylene : 0.273 Kg/hour

inert compounds : 1.336 Kg/hour

ethanol : 0.317 Kg/hour

ETBE : 2.682 Kg/hour

This mixture is distilled in the column 57 which is operated with a temperature of 138°C at the foot and 53°C at the head, and a pressure of 5.5 bar measured at the head. Thus a flow is separated at the head of the column with the following composition:

isobutylene : 0.273 Kg/hour

inert compounds : 1.336 Kg/hour

ethanol : 0.025 Kg/hour

which is recycled to the reactor 54 of the second stage through the line 50.

The product separated at the foot of the column 57 is fed through the line 51 to the column 58 which is operated with a temperature at the foot of 108°C and at the head of 98°C, and at a pressure of 3 bar measured at the head.

At the foot of the column 58, ETBE is recovered through the line 53 (purity about 99%). The products separated at the head of the column are recycled to the reactor 56.

The reactor 54, in addition to the recycled flow through the line 50, is also fed with the flow through the line 41, which consists of fresh ethanol in quantities of 0.902 kg/hour. The reactor 54 is operated with the Amberlyst 15 catalyst in the form of a fixed bed, with the reagents in the liquid phase, at about 70°C, under a pressure of 20 bar and with a space velocity of the reagent mixture of about 6 hour$^{-1}$, and a reaction product is discharged through the line 42 with the following composition:

9

isobutylene : 0.025 Kg/hour

inert compound : 1.336 Kg/hour

ethanol : 0.722 Kg/hour

ETBE : 0.454 Kg/hour

This reaction mixture is distilled in the column 55 which is operated with a temperature at the foot of 112°C and at a head of 23°C and at a pressure of 3 bar measured at the head. Thus a mixture is separated at the foot of the column which consists of:

ethanol : 0.722 Kg/hour

inert compounds : 0.454 Kg/hour

which is conveyed to the reactor 56 through the line 46. The product at the head of the column 55 constitutes the exhausted $C_4$-fraction which contains 1.77% by volume of isobutylene and is discharged through the line 45.

In this test none of the exhausted $C_4$-fraction is recycled to the reactor 54. Moreover a total molar ratio of the methanol to the isobutylene in the feed of about 1/1 is used.

The yield of ETBE, relative to the isobutylene feed, is about 97.8%.

Example 12

This is carried out in a manner similar to Example 11, there being supplied to the reactor 56 through the line 48, 2.458 kg/hour of the fresh $C_4$-fraction and, through the line 47, a recycled flow constituted by:

ethanol : 1.014 Kg/hour

ETBE : 1.146 Kg/hour

The reactor 56 is operated with a space velocity of about 10 hour$^{-1}$ and with the other conditions described in Example 11 and a reaction mixture is discharged through the line 49 having the following composition:

isobutylene : 0.275 Kg/hour

inert compounds : 1.336 Kg/hour

ethanol : 0.318 Kg/hour

ETBE : 2.690 Kg/hour

This reaction mixture is distilled in the columns 57 and 58 as in Example 11, with the production of the following flows:

line 50 (recycle to reactor 54):
isobutylene : 0.275 Kg/hour

inert compounds : 1.336 Kg/hour

ethanol : 0.025 Kg/hour

line 52 (recycle to reactor 56):
ethanol : 0.293 Kg/hour

ETBE : 0.683 Kg/hour

line 53:
ETBE with approximately 99% titre.

To the reactor 54, in addition to the recycled flow through the line 50, there is also fed a flow of fresh ethanol of 0.905 Kg/hour through the line 41, and a recycle flow through the line 44 having the following composition:

isobutylene : 0.010 Kg/hour

inert compounds : 0.544 Kg/hour

The reactor 54 is operated as in Example 11, the height of the catalyst bed being varied so as to achieve a space velocity of 6 hour$^{-1}$.

Under these conditions, a reaction mixture is recovered at the foot of the reactor 54 through the line 42 with the following composition:

isobutylene : 0.029 Kg/hour

inert compounds : 1.880 Kg/hour

ethanol : 0.721 Kg/hour

ETBE : 0.422 Kg/hour

This reaction mixture is distilled in the column 55 as in Example 11, a fraction being obtained at the foot which is conveyed to the reactor 56 through the line 46, and at the head (line 43) a fraction which is in part recycled to the reactor 54 (line 44) and in part is discharged as the exhausted $C_4$-fraction (line 45). This latter has an isobutylene content of about 1.5% by volume.

In this test an overall molar ratio between the ethanol and isobutylene in the feed of about 1/1 is used.

The ratio between the total number of moles of inert, aliphatic hydrocarbon and the number of moles of ether formed under approximately equilibrium conditions in the reactor 54 is about 7.3/1.

The yield of ETBE is, moreover, 98.2%.

Example 13

This is carried out as in Example 11, the reactor 56 being fed through the line 48 with 2.458 Kg/hour of the fresh $C_4$-fraction and through the line 47 with a recycled flow constituted by:

ethanol : 1.014 Kg/hour

ETBE : 1.165 Kg/hour

The reactor 56 is operated with a space velocity of about 10 hour$^{-1}$ and with the other conditions described in Example 11, and a reaction mixture is discharged through the line 49 with the following composition:

isobutylene : 0.277 Kg/hour

inert compounds : 1.336 Kg/hour

ethanol : 0.319 Kg/hour

ETBE : 2.706 Kg/hour

This reaction mixture is distilled in the columns 57 and 58 as in Example 11 with the production of the following flows:

line 50 (recycle to reactor 54):
 isobutylene : 0.277 Kg/hour

 inert compounds : 1.336 Kg/hour

 ethanol : 0.025 Kg/hour

line 52 (recycle to reactor 56):
 ethanol : 0.294 Kg/hour

 ETBE : 0.686 Kg/hour

line 53:
 ETBE  with approximately 99% titre.

To the reactor 54, in addition to the recycled flow through the line 50, is fed a flow of fresh ethanol of 0.911 Kg/hour through the line 41, and, through the line 44 a recycled flow having the following composition:

isobutylene : 0.029 Kg/hour

inert compounds : 2.876 Kg/hour

The reactor 54 is operated as in Example 11, the height of the catalyst bed being varied so as to achieve a space velocity of 6 hour$^{-1}$. Under these conditions, a reaction mixture is recovered at the foot of the reactor 54 through the line 42 with the following composition:

isobutylene : 0.043 Kg/hour

inert compounds : 4.162 Kg/hour

ethanol : 0.720 Kg/hour

ETBE : 0.478 Kg/hour

This reaction mixture is distilled in the column 55 as in Example 11, a fraction being obtained at the foot which is fed to the reactor 56 through the line 46 and a fraction being obtained at the head (line 43) which is in part recycled to the reactor 54 (line 44) and is in part discharged as the exhausted C$_4$-fraction (line 45). This latter has an isobutylene content of about 1% by volume.

In this test an overall molar ratio between the ethanol and the isobutylene in the feed of about 1/1 is used.

The ratio between the total number of moles of inert, aliphatic hydrocarbon and the number of moles of ether formed under approximately equilibrium conditions in the reactor 54 is about 15.7/1.

The yield of ETBE, is moreover, 98.8%.

Example 14

This is carried out as in Example 11, the reactor 56 being fed through the line 48 with 2.458 Kg/hour of the fresh C$_4$-fraction and, through the line 47, with a recycled flow constituted by:

ethanol : 1.014 Kg/hour

ETBE : 1.171 Kg/hour

The reactor 56 is operated with a space velocity of about 10 hour$^{-1}$ and with the other conditions described in Example 11 and a reaction mixture is discharged through the line 49 with the following composition:

isobutylene : 0.277 Kg/hour

inert compounds : 1.336 Kg/hour

ethanol : 0.320 Kg/hour

ETBE : 2.711 Kg/hour

This reaction mixture is distilled in the columns 57 and 58 as in Example 11, with the production of the following flows:

line 50 (recycle to reactor 54):
 isobutylene : 0.277 Kg/hour

 inert compounds : 1.336 Kg/hour

 ethanol : 0.025 Kg/hour

line 52 (recycle to reactor 56):
 ethanol : 0.294 Kg/hour

 ETBE : 0.687 Kg/hour

line 53:
 ETBE  with approximately 99% titre.

To the reactor 54, in addition to the recycled flow through line 50, is fed a flow of fresh ethanol of 0.913 Kg/hour, through the line 41, and, through the line 44, a recycled flow having the following composition:

isobutylene : 0.040 Kg/hour

inert compounds : 4.814 Kg/hour

The reactor 54 is operated as in Example 11, the height of the catalyst being varied so as to achieve a space velocity of 6 hour$^{-1}$. Under these conditions, a reaction mixture is recovered at the foot of the reactor 54 through the line 42 with the following composition:

isobutylene        :   0.051 Kg/hour

inert compounds     :   0.150 Kg/hour

ethanol           :   0.719 Kg/hour

ETBE             :   0.486 Kg/hour

This reaction mixture is distilled in the column 55 as in Example 11, a fraction being obtained at the foot which is conveyed to the reactor 56 through the line 46 and a fraction being obtained at the head (line 43) which is in part recycled to the reactor 54 (line 44) and is in part discharged as the exhausted $C_4$-fraction (line 45). This latter has an isobutylene content of about 0.8% by volume.

In this test, an overall molar ratio of the methanol to the isobutylene in the feed of about 1/1 is used. The ratio of the total number of moles of inert, aliphatic hydrocarbon to the number of moles of ether formed under approximately equilibrium conditions in the reactor 54 is about 22.9/1.

The yield of ETBE is moreover 99%.

In all the Examples 11 to 14 described, only traces of oligomers of isobutylene were present in the etherification products.

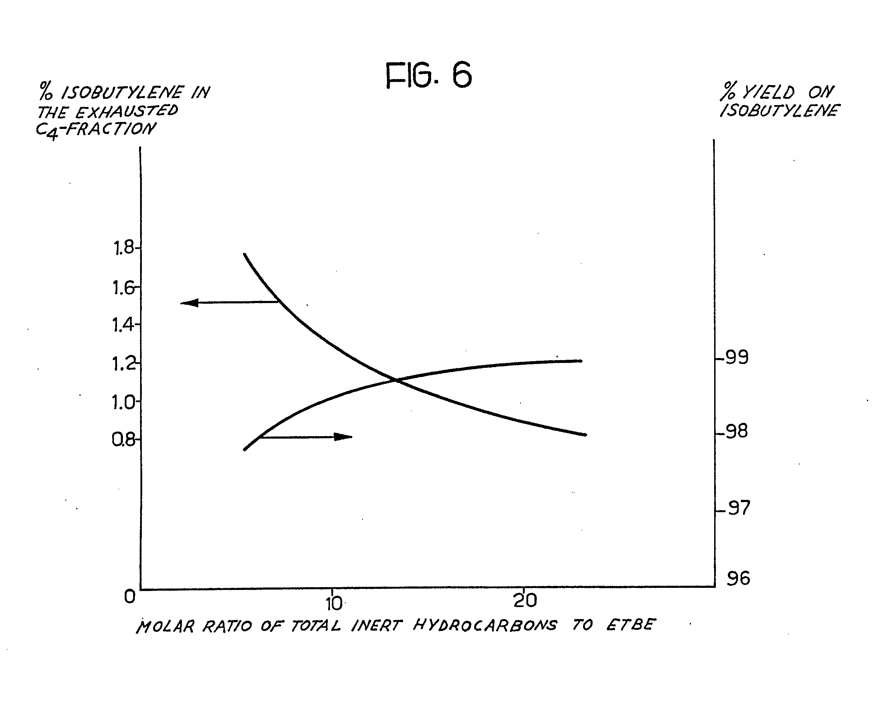

Figure 6 shows graphically the course of the reactions of Example 11 to 14, the molar ratio between the total quantity of inert, aliphatic hydrocarbons and the ETBE formed under approximately equilibrium conditions in the reactor 54 being shown on the abscissa as a function of the percentage by volume of isobutylene in the exhausted $C_4$-fraction discharged, and the yield of ETBE expressed as a molar percentage of the isobutylene supplied.

## Claims

1. Process for the preparation of methyl tert-butyl ether or ethyl tert-butyl ether by the selective reaction of methanol or ethanol respectively with isobutylene contained in a $C_4$-hydrocarbon fraction, in the liquid phase, in the presence of acid catalysts, with a molar ratio of the alcohol to the isobutylene of from 1:1 to 1.25:1, at a temperature of from 40° to 100°C and at a pressure of from 15 to 40 bars, the reaction medium being diluted by an aliphatic hydrocarbon or mixtures of aliphatic hydrocarbons which are liquid under the operating conditions and inert to the other constituents of the reaction medium and which are the recycle product obtained from the reaction mixture after separation of at least the formed ether, characterised in that the recycle product is fed to the reaction medium in an amount to maintain, at the equilibrium, a molar ratio of aliphatic hydrocarbon with the formed ether higher than 7:1.

2. Process according to Claim 1, characterised in that the formed ether is methyl tert-butyl ether and the molar ratio of aliphatic hydrocarbon to the formed ether is from 7:1 to 12:1, at the equilibrium.

3. Process according to Claim 1, characterised in that the formed ether is ethyl tert-butyl ether and the molar ratio of aliphatic hydrocarbon to the formed ether is from 15:1 to 30:1, at the equilibrium.

## Patentansprüche

1. Verfahren zur Herstellung von Methyl-tert.butyläther oder Äthyl-tert.butyläther durch wahlweise Reaktion von Methanol bzw. Äthanol mit in einer $C_4$-Kohlenwasserstoffraktion enthaltenem Isobutylen in flüssiger Phase in Gegenwart von sauren Katalysatoren bei einem molaren Verhältnis des Alkohols zu dem Isobutylen von 1:1 zu 1,25:1 bei einer Temperatur zwischen 40—100°C und einem Druck von 15—40 bar, wobei das Reaktionsmedium mit einem aliphatischen Kohlenwasserstoff oder Mischungen von aliphatischen Kohlenwasserstoffen verdünnt ist, die unter den Arbeitsbedingungen flüssig und mit Bezug auf die anderen Bestandteile des Reaktionsmediums inert sind, und die das aus dem Reaktionsgemisch nach Abtrennung von zumindest dem gebildeten Äther erhaltene Umwälzungsprodukt sind, dadurch gekennzeichnet, daß das Umwälzungsprodukt dem Reaktionsmedium in einem solchen Anteil zugeführt wird, daß bei dem Gleichgewicht ein molares Verhältnis von aliphatischem Kohlenwasserstoff zu dem gebildeten Äther aufrechterhalten wird, das größer als 7:1 ist.

2. Verfahren nach Anspruch 1, dadurch gekennzeichnet, daß der gebildete Äther Methyl-tert.butyläther ist und das molare Verhältnis von aliphatischem Kohlenwasserstoff zu dem gebildeten Äther bei idem Gleichgewicht zwischen 7:1 und 12:1 ist.

3. Verfahren nach Anspruch 1, dadurch gekennzeichnet, daß der gebildete Äther Äthyl-tert.butyläther ist und das molare Verhältnis von aliphatischem Kohlenwasserstoff zu dem gebildeten Äther bei dem Gleichgewicht zwischen 15:1 und 30:1 ist.

## Revendications

1. Procédé pour la préparation d'éther méthyl tert-butylique ou d'éther éthyl tert-butylique par la réaction sélective de méthanol ou d'éthanol avec de l'isobutylène contenu dans une fraction hydrocarbonée en $C_4$ en phase liquide, en présence de catalyseurs acides, avec un rapport molaire de l'alcool à l'isobutylène de 1:1 à 1,25:1 à une température de 40° à 100°C et à une pression de 15 à 40 bars, le milieu réactionnel étant dilué par un hydrocarbure aliphatique ou des mélanges d'hydrocarbures aliphatiques qui sont liquides sous les conditions opératoires et inertes vis-à-vis des autres constituants du milieu réactionnel et qui sont le produit de recyclage obtenu à partir du milieu réactionnel après séparation d'au moins l'éther formé, caractérisé par le fait que le produit de recyclage est introduit dans le milieu réactionnel en une quantité pour maintenir, à l'équilibre, un rapport molaire d'hydrocarbone aliphatique avec l'éther formé supérieur à 7:1.

2. Procédé selon la revendication 1, caractérisé par le fait que l'éther formé est l'éther méthyl tert-butylique et que le rapport molaire de l'hydrocarbure aliphatique à l'éther formé est 7:1 à 12:1, à l'équilibre.

3. Procédé selon la revendication 1, caractérisé par le fait que l'éther formé est l'éther éthyl tert-butylique et que le rapport molaire de l'hydrocarbure aliphatique à l'ether formé est de 15:1 à 30:1, à l'équilibre.

# FIG. 1

# FIG. 2

% ISOBUTYLENE
IN THE EXHAUSTED
C$_4$-FRACTION

% YIELD
ON
ISOBUTYLENE

MOLAR RATIO OF TOTAL INERT HYDROCARBONS TO MTBE

# FIG. 3

FIG. 4

% ISOBUTYLENE IN THE EXHAUSTED C₄-FRACTION

% YIELD ON ISOBUTYLENE

MOLAR RATIO OF TOTAL INERT HYDROCARBONS TO ETBE

0 071 032

# FIG. 5

# FIG. 6

% ISOBUTYLENE IN
THE EXHAUSTED
C₄-FRACTION

% YIELD ON
ISOBUTYLENE

MOLAR RATIO OF TOTAL INERT HYDROCARBONS TO ETBE

0 071 032